# EUROPEAN PATENT APPLICATION

(11) **EP 1 987 814 A1**
(43) Date of publication of application: **05.11.2008**
(21) Application number: 07290540.9
(22) Date of filing: 30.04.2007
(51) Int. Cl.: A61K 9/00, A61K 9/20, A61K 31/567

(54) **Mifepristone pharmaceutical compositions and their methods of preparation**

(71) Applicant: Exelgyn, 75007 Paris (FR)
(72) Inventor: Prinderre, Pascal, 13004 Marseille (FR); Paris, Jean-Pierre, 78120 Sonchamp (FR)
(74) Representative: Hirsch & Associés

(57) **Abstract**

A mifepristone tablet composition comprising more than 70% by weight of mifepristone and method of preparation.

## Description

### FIELD OF THE INVENTION

The invention relates to novel mifepristone compositions and novel methods for preparing them.

### BACKROUND OF THE INVENTION

Mifepristone, also known as RU486 has the chemical formula 11β-[p-(Dimethylamino)phenyl]-17β-hydroxy-17-(1-propynyl)estra-4,9-dien-3-one, and is an important antiprogestin that effectively and safely terminates early pregnancy, when used in conjunction with a prostaglandin. It also used for cervix dilation before subsequent termination of pregnancy during the first trimester by vacuum aspiration, preparation of the cervix for termination of pregnancy for medical reasons after the first trimester, and induction of labor when the fetus has died in the uterus.

Mifepristone is commercially available in a dose of 3 tablets of 200 mg under the trade name Mifegyne^{®}. For the interruption of pregnancy a prostaglandin is administered 36 to 48 hours afterwards. Mifegyne^{®} is currently produced using a wet granulation method employing a water/alcohol mixture. The composition also comprises starch.

Other dosages and formulations of mifepristone have been disclosed in the prior art.

WO-A-200422067 discloses a semi-solid skeleton composition including 0.5-5 wt% mifepristone, 1-50 wt% surfactant with HLB greater than 12 and 45-98.5 wt% semi-solid skeleton carrier. The composition has good leaching performance, powerful early pregnancy resisting effect, rat ED50 as high as 17 times that of the marketed tablet and dog's bioavailability as high as 5 times that of the marketed tablet. Central double-blind clinical research shows that the present invention has threefold clinical effect as the marketed tablet. The mifepristone capsule of the present invention has the advantages of small dosage, less negative effect and high bioavailability.

CN-A-1311000 discloses a mifepristone capsule which contains mifepristone and auxiliary materials including propylene glycol, ethyl alcohol, Tween^{®}20, polyethylene glycol 400, etc., capsuled with gelatin. The mifepristone solution can also be composed of mifepristone, linolic acid, Tween^{®}80, vitamin E, etc.. The advantages reported are: high bioavailability, low dose, less adverse effect.

CN-A-1218665 discloses a mifepristone preparation which consists of mifepristone, a pharmaceutically acceptable surfactant and medicinal additive, and the said surfactant is selected from non-ionic surfactant and ionic surfactant. It also relates to the preparation process of the said preparation and its application in preparing medicine for resisting early-stage pregnancy abortion and medicine for emergency contraception and medicine for curing uterine, endometriosis, breast cancer and other gynopathy. The preparation has the advantages of high biological utilization and reduced side effect, and its preparation process is said to be simple and convenient.

WO-A-9808471 discloses the delivery of mifepristone in low dose amounts either vaginally or in the uterus, where mifepristone is used as a contraceptive.

CN-A-1634078 discloses a composition and tablet of anorethidrane dipropionate and mifepristone wherein the compound comprises (by weight ratio) 6 parts Mifepristone, 1 part anorethidrane dipropionate and auxiliary solvent, wherein the mifepristone and anorethidrane dipropionate are highly soluble.

There is still a need for new mifepristone compositions and new processes for making the same.

### SUMMARY OF THE INVENTION

The invention thus provides a mifepristone tablet composition comprising more than 70% by weight of mifepristone.

The invention also provides a mifepristone tablet composition comprising 600 mg of mifepristone.

According to one embodiment, the mifepristone tablet comprises more than 70% by weight of mifepristone.

The invention also provides a mifepristone tablet comprising less than 15% by weight starch.

According to yet another embodiment, the mifepristone tablet comprises less than 5% by weight starch, and preferably is substantially devoid of starch.

According to yet another embodiment, the mifepristone tablet comprises the following ingredients, by weight:

| | wt% |
|---|---|
| Mifepristone, micronized | 40-80 |
| Microcrystalline cellulose | 10-25 |
| Binder | 3-10 |
| Starch | < 15% |
| Diluent | Up to 15% |
| Disintegrating agent | 0.5-5 |
| Lubricant | 0.5-5 |
| Antistatic | 0-3% |

According to yet another embodiment, the mifepristone tablet comprises the following ingredients, by weight:

| | wt% |
|---|---|
| Mifepristone, micronized | 63-80 |
| Microcrystalline cellulose | 10-20 |
| Binder | 3-8 |
| Starch | About 0 |
| Diluent | Up to 5 |
| Disintegrating agent | 1-3 |
| Lubricant | 1-3 |
| Antistatic | 0-2 |

According to yet another embodiment, the mifepristone tablet comprises the following ingredients, by weight:

| | wt% |
|---|---|
| Mifepristone, micronized | 70-80 |
| Microcrystalline cellulose | 13-18 |
| Binder | 4-6 |
| Starch | About 0 |
| Diluent | <1 |
| Disintegrating agent | 1,5-2,5 |
| Lubricant | 1,5-2,5 |
| Antistatic | 0-1,5 |

According to yet another embodiment, the binder is selected from the group consisting of polyvinylpyrrolidone, polyvinylpyrrolidone-vinyl acetate copolymer, polyvinylalcohol, hydroxypropylcellulose, hydroxymethylcellulose, hydroxypropylmethylcellulose, methylcellulose, ethylcellulose, gelatin, and mixtures thereof.

According to yet another embodiment, the diluent is selected from the group consisting of lactose, saccharose, mannitol, and mixtures thereof.

According to yet another embodiment, the disintegrating agent is selected from the group consisting of cross-linked polyvinylpyrrolidone, croscarmellose and mixtures thereof.

According to yet another embodiment, the lubricant is selected from the group consisting of talc, magnesium stearate and glyceryl behenate, and mixtures thereof.

According to yet another embodiment, the antistatic agent is anhydrous colloidal silica.

The invention also provides a mifepristone tablet having a dissolution, as measured using the type II paddle method at 100 rpm according to the US Pharmacopoeia, in a dissolution medium comprised of 1000 ml HCl 0.01M, as follows:
- at 5 minutes between 45 and 80%;
- at 10 minutes between 55 and 85%;
- at 15 minutes between 65 and 90%; and
- at 20 minutes more than 75%.

According to an embodiment, the mifepristone tablet has a dissolution, as measured using the type II paddle method at 100 rpm according to the US Pharmacopoeia, in a dissolution medium comprised of 1000 ml HCl 0.01M, as follows:
- at 5 minutes between 50 and 70%;
- at 10 minutes between 60 and 80%;
- at 15 minutes between 70 and 90%; and
- at 20 minutes more than 85%.

According to yet another embodiment, the mifepristone compositions as recited in three tables above have a dissolution, as measured using the type II paddle method at 100 rpm according to the US Pharmacopoeia, in a dissolution medium comprised of 1000 ml HCl 0.01M, as follows:
- at 5 minutes between 45 and 80%;
- at 10 minutes between 55 and 85%;
- at 15 minutes between 65 and 90%; and
- at 20 minutes more than 75%.

According to yet another embodiment, the mifepristone compositions as recited in three tables above have a dissolution, as measured using the type II paddle method at 100 rpm according to the US Pharmacopoeia, in a dissolution medium comprised of 1000 ml HCl 0.01M, as follows:
- at 5 minutes between 50 and 70%;
- at 10 minutes between 60 and 80%;
- at 15 minutes between 70 and 90%; and
- at 20 minutes more than 85%.

The invention also provides a process for manufacturing mifepristone tablets, comprising the following steps:
(i) mixing mifepristone together with inert excipients, binder, and optionally further excipients and a sufficient amount of water to form granulates, in the absence of organic solvent;
(ii) optionally sieving into granulates;
(iii) drying said granulates;
(iv) mixing the dry granulates with compression aids; and
(v) compressing into tablets.

In one embodiment, the compression aids are disintegrant agent and lubricant.

In yet another embodiment, the process further comprises a sieving step after the drying step (iii).

In yet another embodiment, step (i) comprises two substeps, (ia) dry mixing and (ib) granulating with water.

The invention also provides a process for manufacturing mifepristone tablets, comprising the following steps:
(i) dry mixing mifepristone together with the excipients including a binder; and
(ii) compressing into tablets.

In one embodiment, step (i) comprises two substeps, (ia) dry mixing all excipients except lubricant and (ib) mixing the lubricant afterwards.

In another embodiment, the binder comprises vinylpyrrolidone as a monomer.

In yet another embodiment, the binder comprises copovidone.

In yet another embodiment, the binder has a particle size d90 of about 15-20 µm.

In one embodiment, the direct compression process is suited for the manufacture of a mifepristone composition comprising the following ingredients, by weight:

| | wt% |
|---|---|
| Mifepristone, micronized | 20-80 |
| Microcrystalline cellulose | 10-50 |
| Binder | 2-10 |
| Starch | < 15% |
| Diluent | Up to 25% |
| Disintegrating agent | 0.5-10 |
| Lubricant | 0.5-5 |
| Antistatic | 0-3% |

In another embodiment, the direct compression process is suited for the manufacture of a mifepristone composition comprising the following ingredients, by weight:

| | wt% |
|---|---|
| Mifepristone, micronized | 50-80 |
| Microcrystalline cellulose | 10-35 |
| Binder | 2-9 |
| Starch | About 0 |
| Diluent | Up to 15 |
| Disintegrating agent | 0,5-3 |
| Lubricant | 1-3 |
| Antistatic | 0-2 |

In yet another embodiment, the direct compression process is suited for the manufacture of a mifepristone composition comprising the following ingredients, by weight:

| | wt% |
|---|---|
| Mifepristone, micronized | 60-80 |
| Microcrystalline cellulose | 10-20 |
| Binder | 2-8 |
| Starch | About 0 |
| Diluent | <5 |
| Disintegrating agent | 0,5-1,5 |
| Lubricant | 1-2 |
| Antistatic | 0-1 |

The processes of the invention are especially suited for the manufacture of the compositions of the invention as depicted above, especially in the tables and according to the dissolution medium.

The invention aims at providing compositions and methods which brings one or more of the following advantages:
- The compositions are bioequivalent or even have better bioavailability, when compared to the existing marketed compositions such as Mifegyne^{®}.
- The mifepristone compositions are easier and safer to manufacture, compared to the existing composition, which is manufactured by a process of wet granulation. In keeping with European regulations there is currently a need to reduce to a minimum the amount of volatile organic compounds used in the manufacture of all pharmaceutical compounds. Thus, a method for the manufacture of a mifepristone with essentially no organic solvent present is advantageous.
- A single dosage tablet of 600 mg of mifepristone, and/or containing least 70% of active ingredient, would be highly advantageous compared to the current dosage form of 3 times a 200 mg tablet. Specifically, the emetic effect of taking one tablet would be considerably less than when taking three and the general facility of administering the treatment would be improved. Also, given the current density of mifepristone, there is a challenge to manufacture compositions and especially tablets with high active contents.
- The mifepristone compositions are acceptable to the maximum of women in terms of the allergic and metabolic tolerance.

### BRIEF DESCRIPTION OF THE DRAWINGS

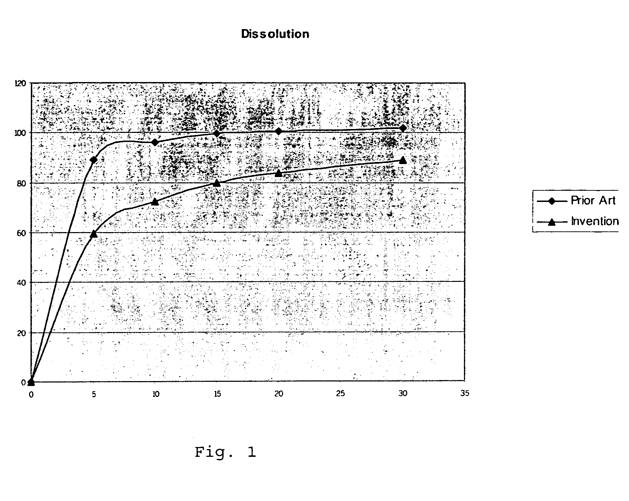

In the drawings, fig. 1 is the dissolution curve of the product according to one embodiment of the invention and the prior art product.

### DETAILED DESCRIPTION OF EMBODIMENTS OF THE INVENTION

The invention relates to novel compositions and novel galenic formulations of mifepristone, the antiprogestin used to medically terminate pregnancy, and for dilation of the cervix. The invention also relates to the methods for the preparation of such novel galenic forms of mifepristone.

In one embodiment, the invention is about a tablet containing 600 mg of mifepristone and/or more than 70% of mifepristone. As indicated, having one tablet only will avoid emetic effect of the drug. Having more than 70% of active is quite surprising, given the bulk density of mifepristone, which is about 0.2. High drug content tablets may contain 600 mg or 200 mg.

The mifepristone compositions currently on the market contain 200 mg of active ingredient, whereas the prescribed does for effective pregnancy termination is 600 mg. Therefore, the current treatment entails taking three tablets of 200 mg mifepristone. The advantage of the composition with 600 mg mifepristone is that the patient is required to take only one tablet, rather than three, for the treatment desired. As well as reducing the stress levels of the patient having to take the tablets, any emetic effect associated with swallowing the tablets is diminished by taking one compared to three of them.

In another embodiment, the invention provides a composition free of starch. Another aspect of the invention is thus that the galenic forms of the invention are essentially free from (maize) starch, i.e. substantially free of gluten. This advantage of this reduction in the amount of or complete absence of (maize) starch in the formulation is that the tablet may be taken by patients suffering from celiac disease. As this disease affects a significant amount of the world's population the advantage of the current formulation is significant.

In yet another embodiment, the invention provides a composition comprising the following components, expressed in wt%:

| | | Pref. | Most pref |
|---|---|---|---|
| Mifepristone, micronized | 40-80 | 63-80 | 70-80 |
| Microcrystalline cellulose | 10-25 | 10-20 | 13-18 |
| Binder | 3-10 | 3-8 | 4-6 |
| (Maize) Starch | < 15% | About* 0 | About* 0 |
| Diluent | Up to 15% | Up to 5% | <1 |
| Disintegrating agent | 0.5-5 | 1-3 | 1,5-2,5 |
| Lubricant | 0.5-5 | 1-3 | 1,5-2,5 |
| Antistatic | 0-3% | 0-2 | 0-1,5 |

| | | | |
|---|---|---|---|
| * indicates none is added; but the composition can be manufactured in a plant using such starch. | | | |

Mifepristone, and especially micronized mifepristone, is a known active ingredient. The d50 of the miocronized form is generally less than 30 µm, preferably less than 20 µm. This active ingredient may represent 40 to 80%, preferably between 63 and 80%, and most preferably between 70 and 80% by weight of the tablet. The ingredients according to invention have the following proportions: mifepristone 40-80%, excipients 20-60%. For a 600 mg tablet, the weight may vary e.g. from 700 to 900 mg, typically it is about 800 mg.

Microcrystalline cellulose is also a well known component. It is available under the trade name Avicel^{®}, especially Avicel^{®} PH101.

The starch content is preferably less than 15%, more preferably less than 5%, and advantageously the composition is substantially free of starch.

Excipient includes any of the following components binder, diluent, disintegrating agent, lubricant and anti-static agent and diluent and optionally other auxiliary agents, such as surfactants.

The expression "binder" in the invention should be taken to mean any high molecular weight substance (greater, for example, than 300) having sufficient affinity towards water to dissolve therein and form a gel, allowing the final obtention of a film. Examples of such polymers are polyvinylpyrrolidone, polyvinylalcohol, hydroxypropylcellulose, hydroxymethylcellulose, hydroxypropylmethylcellulose, methylcellulose, ethylcellulose, gelatin, etc. or any other film-forming polymer known in the art (such as the polymers disclosed in "Handbook of Pharmaceutical excipients", 2nd Ed., 1994, American Pharmaceutical Association, Washington, ISBN 0 91730 66 8, by Wade A., Weller PJ.). Polymer blends are also suitable. The preferred binder is PVP.

In the framework of this invention, the expression "diluent" means any excipient which acts to dilute the formulation without undergoing a chemical reaction with the formulation components. A diluent of the invention includes generally inert carriers or vehicles, be it crystalline or amorphous. Examples of such diluents are derivatives of sugars, such as lactose, saccharose, mannitol, etc., and mixtures thereof. Hydrolyzed starch (malto-dextrine) can be used, preferably in low amounts.

In the framework of this invention, the expression "disintegrating agent" means any excipient which exerts an action of disintegrating when the tablet is in an aqueous environment, i.e. which will exert an internal pressure leading to breaks in the tablet and ultimately to its disintegration. Examples of disintegrating agents of the invention are cross-linked polyvinylpyrrolidone, croscarmellose, and mixtures thereof.

In the framework of this invention, the expression "lubricant" means any excipient which improves the flowability of the composition. Examples of lubricants are talc, magnesium stearate and glyceryl behenate, and mixtures thereof.

In the framework of this invention, the expression "anti-static agent" means any excipient which exerts an anti-static action. Examples of antistatic agents are anhydrous colloidal silica (which may also act as a disintegrant).

According to another embodiment of the invention, the compositions of the invention have a specific dissolution profile. The galenic forms of mifepristone according to one embodiment of the invention have a dissolution, as measured using the type II paddle method at 100 rpm according to the US Pharmacopoeia, in a dissolution medium comprised of 1000 ml HCl 0.01M,
- at 5 minutes between 45 and 80%, preferably between 50 and 70%;
- at 10 minutes between 55 and 85%, preferably between 60 and 80%;
- at 15 minutes between 65 and 90%, preferably between 70 and 90%;
- at 20 minutes more than 75%, preferably more than 85%.

Figure 1 shows the dissolution profiles of the mifepristone galenic form of the invention (square) compared to that of the prior art (lozenge). The dissolution profile of the current invention provides for an improved bioavailability compared to the galenic form of the prior art. This is due to a relatively smoother dissolution profile of the galenic forms in the present invention, compared to the prior art.

According to yet a further embodiment of the invention, there is provided a process for the manufacture of a mifepristone composition, whereby organic solvents are avoided. The composition according to the invention is prepared by a novel "semi-moist" method. With this process, the use of volatile organic solvents such as alcohols is avoided, thus the method is safer to carry out than the wet granulation process of the prior art and also it conforms to current European regulations on use of VOCs.

The "semi-moist" method uses water only as the granulating liquid. This process comprises the following steps:
(i) mixing mifepristone together with inert excipients, binder, and optionally further excipients and a sufficient amount of water to form granulates, in the absence of organic solvent;
(ii) optionally sieving into granulates;
(iii) drying said granulates;
(iv) mixing the dry granulates with compression aids; and
(v) compressing into tablets.

Step (i) may comprise two substeps, such as (ia) dry mixing the components followed by (ib) mixing with water to provide granulates. The mixing step with water may also be carried out stepwise, i.e. water can be added by parts (typically in two halves). Mixing time for step (i) is typically from one to ten minutes. Typically, in step (i), micronized mifepristone, microcrystalline cellulose, binder such as PVP and colloidal silica are mixed and then water is added to form granulate. The amount of water is typically from 10 to 45%, based on the weight of the dry ingredients.

Fluidized bed granulation is also an alternative method for step (i). In this case, the amount of water will be dependent upon the operative granulation conditions, as will appreciated by the skilled addressee.

Step (ii) is optional but preferred in the instant invention. The sieve size can vary but is generally from 1 and 5mm, preferably about 4 mm.

Step (iii) is a drying step. Granulates are spread on a plate, for a thickness of typically less than 5 cm, and then placed in an oven. The temperature and time are sufficient to dry the granulates to a humidity level below 3%, typically from 0,5 to 2,5%. The temperature is typically from 40 to 80°C; exemplary values are 45-60°C. Time is generally from 1 to 10 hours, depending on the water amount. Typical times are 3-6 hours.

An optional step of sieving can be carried out at the end of the drying step (typically sieving size is 0,2-2 mm, typically 0,625-1,5 mm or 0,8-1,5 mm), especially to calibrate the granulates. An optional sieving step may also be carried out during the drying step.

Step (iv) comprises mixing the dry granulates with compression aids. An optional sieving step at the end of the mixing step can be carried out (typically sieving size is 0,8-1,5 mm). Compression aids typically comprise the disintegrant and the lubricant.

Step (v) is the final compression step into tablets. This is done on a classical tabletting machine. This step may be generally carried out using a rotary tablet press, e.g. with a D15R20 punch. The resulting tablets generally have an average mass of 780-815 mg, preferably 800 mg, a diameter of 12-18mm, preferably 15 mm, a thickness of 4-8 mm, preferably 6 mm, with a hardness of 75-95N, preferably 90N and a residual humidity of less than or equal to 2.5%, although a higher moisture level may be acceptable.

According to yet a further embodiment of the invention, there is provided a further process for the manufacture of a mifepristone composition, whereby organic solvents are avoided. The composition according to the invention is prepared by a novel direct compression method. With this process as well, the use of volatile organic solvents such as alcohols is avoided, thus the method is safer to carry out than the wet granulation process of the prior art and also it conforms to current European regulations on use of VOCs.

The novel direct compression uses a specific binder, which comprises vinylpyrrolidone as a monomer. A preferred binder is a copolymer with vinyl acetate, known as copovidone. Copovidone is disclosed in the Handbook of Pharmaceutical Excipients, 2004. Preferably the particle size of the binder is a fine grade, e.g. has a particle size d90 of about 15-20 µm.

Further compression aids, as disclosed above, are also used in the direct compression method. Diluents, lubricants, glidants, disintegrants, and the like may be used.

A typical formulation would be:

| | wt% |
|---|---|
| Mifepristone, micronized | 20-80 |
| Microcrystalline cellulose | 10-50 |
| Binder | 2-10 |
| Starch | < 15% |
| Diluent | Up to 25% |
| Disintegrating agent | 0.5-10 |
| Lubricant | 0.5-5 |
| Antistatic | 0-3% |

A preferred formulation would be

| | wt% |
|---|---|
| Mifepristone, micronized | 50-80 |
| Microcrystalline cellulose | 10-35 |
| Binder | 2-9 |
| Starch | About 0 |
| Diluent | Up to 15 |
| Disintegrating agent | 0,5-3 |
| Lubricant | 1-3 |
| Antistatic | 0-2 |

A more preferred formulation would be

| | wt% |
|---|---|
| Mifepristone, micronized | 60-80 |
| Microcrystalline cellulose | 10-20 |
| Binder | 2-8 |
| Starch | About 0 |
| Diluent | <5 |
| Disintegrating agent | 0,5-1,5 |
| Lubricant | 1-2 |
| Antistatic | 0-1 |

The direct compression process of the invention comprises the following steps:
(i) dry mixing mifepristone together with the excipients; and
(ii) compressing into tablets.

Lubricant may be added separately after a first mixing step. In that step all ingredients but the lubricant are mixed in a first substep and the lubricant is then mixed in a second substep. Mixing times are typically from one to ten minutes.

The compression step is similar to the one disclosed above for the semi-moist process.

### EXAMPLES

The following examples illustrate the invention without limiting it.

### Example 1.

The following formulation is prepared.

| | wt% | mg |
|---|---|---|
| Mifepristone, micronized | 75 | 600 |
| Microcrystalline cellulose | 15 | 120 |
| PVP | 5 | 40 |
| Colloidal silica | 1 | 8 |
| Crospovidone | 2 | 16 |
| Talc | 1 | 8 |
| Magnesium stearate | 1 | 8 |
| Total | 100 | 800 |

Micronized mifepristone (having a particle size such that 96% of the material has a size below 15 µm), microcrystalline cellulose, PVP and colloidal silica are mixed in dry conditions. Mixing time is for about 2 minutes.

Then water is added (15,625% based on dry composition, corresponding to 125 mg) is added and mixing is continued for about 2 minutes. A second water amount of 15,625% is added in two increments (half each time), after about one minute. Between each addition of water the mixing is continued for 5 minutes.

The resulting granulate is then sieved on a proper sieve (4 mm).

These granulates are then laid on a plate (thickness of about 1-2 cm) and placed in an oven at a temperature of about 45°C until a residual humidity is between 1,5 and 2,5%. A final 60°C drying stage may be applied. An intermediate sieving may be carried out at a sieving size of 2,5 mm. A final sieving at a size of 1,5 mm is then performed.

Dry granulates are mixed with crospovidone, talc, magnesium stearate. An intermediate sieving at a size of 1 mm may be carried out.

The mass is then tabletted on a tabletting machine equipped with D15R20 punches. The final hardness is about 90N.

Dissolution tests are carried out, in an apparatus according to the US Pharmacopea, XXIV, type II paddle, at 100 rpm, in a dissolution medium comprised of 1000 ml of HCl 0.1N.

The results (expressed in dissolved fraction in %) are represented in the attached figure 1. Results show that the release in the case of the invention is less marked than the prior art product, leading to better bioavailability.

### Example 2.

The following formulation is prepared.

| | wt% | Mg |
|---|---|---|
| Mifepristone, micronized | 64 | 200 |
| Microcrystalline cellulose | 27.04 | 84.50 |
| Copovidone | 5 | 15.63 |
| Colloidal silica | 0.86 | 2.69 |
| Crospovidone | 1.5 | 4.69 |
| Talc | | |
| Magnesium stearate | 1.6 | 5 |
| Total | 100 | 312.51 |

All ingredients except the lubricant are first mixed. The lubricant is then added and mixed. The mixture is thend fed to a rotating direct compression apparatus.

## Claims

1. A mifepristone tablet composition comprising more than 70% by weight of mifepristone.

2. A mifepristone tablet composition comprising 600 mg of mifepristone.

3. The mifepristone tablet of claim 2 comprising more than 70% by weight of mifepristone.

4. A mifepristone tablet comprising less than 15% by weight starch.

5. The mifepristone tablet of claim 4 comprising less than 5% by weight starch, and preferably is substantially devoid of starch.

6. The mifepristone tablet of claim 4, comprising the following ingredients, by weight:
| | wt% |
|---|---|
| Mifepristone, micronized | 40-80 |
| Microcrystalline cellulose | 10-25 |
| Binder | 3-10 |
| Starch | < 15% |
| Diluent | Up to 15% |
| Disintegrating agent | 0.5-5 |
| Lubricant | 0.5-5 |
| Antistatic | 0-3% |

7. The mifepristone tablet of claim 6 comprising the following ingredients, by weight:
| | wt% |
|---|---|
| Mifepristone, micronized | 63-80 |
| Microcrystalline cellulose | 10-20 |
| Binder | 3-8 |
| Starch | About 0 |
| Diluent | Up to 5 |
| Disintegrating agent | 1-3 |
| Lubricant | 1-3 |
| Antistatic | 0-2 |

8. The mifepristone tablet of any one of claims 1 to 7, comprising the following ingredients, by weight:
| | wt% |
|---|---|
| Mifepristone, micronized | 70-80 |
| Microcrystalline cellulose | 13-18 |
| Binder | 4-6 |
| Starch | About 0 |
| Diluent | <1 |
| Disintegrating agent | 1,5-2,5 |
| Lubricant | 1,5-2,5 |
| Antistatic | 0-1,5 |

9. The mifepristone tablet of any one of claims 6 to 8, wherein the binder is selected from the group consisting of polyvinylpyrrolidone, polyvinylpyrrolidone-vinyl acetate copolymer, polyvinylalcohol, hydroxypropylcellulose, hydroxymethylcellulose, hydroxypropylmethylcellulose, methylcellulose, ethylcellulose, gelatin, and mixtures thereof.

10. The mifepristone tablet of any one of claims 6 to 9, wherein the diluent is selected from the group consisting of lactose, saccharose, mannitol, and mixtures thereof.

11. The mifepristone tablet of any one of claims 6 to 10, wherein the disintegrating agent is selected from the group consisting of cross-linked polyvinylpyrrolidone, croscarmellose and mixtures thereof.

12. The mifepristone tablet of any one of claims 6 to 11, wherein the lubricant is selected from the group consisting of talc, magnesium stearate and glyceryl behenate, and mixtures thereof.

13. The mifepristone tablet of any one of claims 6 to 12, wherein the antistatic agent is anhydrous colloidal silica.

14. A mifepristone tablet having a dissolution, as measured using the type II paddle method at 100 rpm according to the US Pharmacopoeia, in a dissolution medium comprised of 1000 ml HCl 0.01M, as follows:
- at 5 minutes between 45 and 80%;
- at 10 minutes between 55 and 85%;
- at 15 minutes between 65 and 90%; and
- at 20 minutes more than 75%.

15. The mifepristone tablet of claim 14, having a dissolution, as measured using the type II paddle method at 100 rpm according to the US Pharmacopoeia, in a dissolution medium comprised of 1000 ml HCl 0.01M, as follows:
- at 5 minutes between 50 and 70%;
- at 10 minutes between 60 and 80%;
- at 15 minutes between 70 and 90%; and
- at 20 minutes more than 85%.

16. The mifepristone tablet of any one of claims 1 to 13, having a dissolution, as measured using the type II paddle method at 100 rpm according to the US Pharmacopoeia, in a dissolution medium comprised of 1000 ml HCl 0.01M, as follows:
- at 5 minutes between 45 and 80%;
- at 10 minutes between 55 and 85%;
- at 15 minutes between 65 and 90%; and
- at 20 minutes more than 75%.

17. The mifepristone tablet of claim 16, having a dissolution, as measured using the type II paddle method at 100 rpm according to the US Pharmacopoeia, in a dissolution medium comprised of 1000 ml HCl 0.01M, as follows:
- at 5 minutes between 50 and 70%;
- at 10 minutes between 60 and 80%;
- at 15 minutes between 70 and 90%; and
- at 20 minutes more than 85%.

18. A process for manufacturing mifepristone tablets, comprising the following steps:
(i) mixing mifepristone together with inert excipients, binder, and optionally further excipients and a sufficient amount of water to form granulates, in the absence of organic solvent;
(ii) optionally sieving into granulates;
(iii) drying said granulates;
(iv) mixing the dry granulates with compression aids; and
(v) compressing into tablets.

19. The process of claim 18, wherein the compression aids are disintegrant agent and lubricant.

20. The process of claim 18 or 19, further comprising a sieving step after the drying step (iii).

21. The process of any one of claims 18 to 20, wherein step (i) comprises two substeps, (ia) dry mixing and (ib) granulating with water.

22. A process for manufacturing mifepristone tablets, comprising the following steps:
(i) dry mixing mifepristone together with the excipients including a binder; and
(ii) compressing into tablets.

23. The process of claim 22, wherein step (i) comprises two substeps, (ia) dry mixing all excipients except lubricant and (ib) mixing the lubricant afterwards.

24. The process of claim 22 or 23, wherein the binder comprises vinylpyrrolidone as a monomer.

25. The process of claim 24, wherein the binder comprises copovidone.

26. The process of any one of claims 22 to 25, wherein the binder has a particle size d90 of about 15-20 *µ*m.

27. The process of any one of claims 22 to 26, for the manufacture of a mifepristone composition comprising the following ingredients, by weight:
| | wt% |
|---|---|
| Mifepristone, micronized | 20-80 |
| Microcrystalline cellulose | 10-50 |
| Binder | 2-10 |
| Starch | < 15% |
| Diluent | Up to 25% |
| Disintegrating agent | 0.5-10 |
| Lubricant | 0.5-5 |
| Antistatic | 0-3% |

28. The process of any one of claims 22 to 26, for the manufacture of a mifepristone composition comprising the following ingredients, by weight:
| | wt% |
|---|---|
| Mifepristone, micronized | 50-80 |
| Microcrystalline cellulose | 10-35 |
| Binder | 2-9 |
| Starch | About 0 |
| Diluent | Up to 15 |
| Disintegrating agent | 0,5-3 |
| Lubricant | 1-3 |
| Antistatic | 0-2 |

29. The process of any one of claims 22 to 26, for the manufacture of a mifepristone composition comprising the following ingredients, by weight:
| | wt% |
|---|---|
| Mifepristone, micronized | 60-80 |
| Microcrystalline cellulose | 10-20 |
| Binder | 2-8 |
| Starch | About 0 |
| Diluent | <5 |
| Disintegrating agent | 0,5-1,5 |
| Lubricant | 1-2 |
| Antistatic | 0-1 |

30. The process of any one of claims 18 to 26 for the manufacture of a composition of any one of claims 1 to 17.
